# EUROPEAN PATENT SPECIFICATION

(11) **EP 3 970 670 B1**
(45) Date of publication and mention of the grant of the patent: **17.04.2024**
(21) Application number: 20805429.6
(22) Date of filing: 12.05.2020
(51) Int. Cl.: A61F 2/54, A61F 2/58, A61F 2/70

(54) **PROSTHESIS DIGIT MEMBER WITH SPIROID REDUCER AND MODULAR DESIGN OF UPPER LIMB PROSTHESIS**
MIT EINEM SPIROIDMINDERER VERSEHENES PROTHESENFINGERELEMENT UND MODULARER AUFBAU EINER ARMPROTHESE
DOIGT DE PROTHÈSE AVEC RÉDUCTEUR SPIROÏDE ET STRUCTURE MODULAIRE DE PROTHÈSE D'EXTRÉMITÉ SUPÉRIEURE

(30) Priority: 13.05.2019 RU 2019114311
(43) Date of publication of application: 23.03.2022
(73) Proprietor: Motorica Limited Liability Company, Moscow, 121205 (RU)
(72) Inventor: SHIRIAZDANOV, Konstantin Valerievich, Moscow, 105215 (RU)
(74) Representative: Spengler, Robert
(86) International application number: PCT/RU2020/000218
(87) International publication number: WO 2020/231294

(56) References cited:
- WO-A1-03/017876
- CN-A- 107 803 849
- US-A- 3 509 583
- US-A- 5 888 246
- US-A1- 2005 021 154
- US-A1- 2010 005 918
- US-A1- 2013 030 550
- ABADJIEV VALENTIN ET AL: "Some principles of mathematical modelling and computer synthesis of hyperboloid gears with a conjugate linear tooth contact", 2014 INTERNATIONAL CONFERENCE ON INFORMATION SCIENCE, ELECTRONICS AND ELECTRICAL ENGINEERING, IEEE, vol. 1, 26 April 2014 (2014-04-26), pages 265-270, XP032672690, DOI: 10.1109/INFOSEEE.2014.6948111 ISBN: 978-1-4799-3196-5 [retrieved on 2014-11-05]

## Description

### FIELD OF INVENTION

The invention belongs to the field of medical engineering, more particularly to prosthetics, and bio-electrical prosthetics, namely prosthetics of upper limbs. In particular, this invention describes the technical result of the invention consisting of a design of an upper limb prosthesis and a wrist of an upper limb bio-electrical prosthesis.

### GLOSSARY

Prosthesis means an artificial replacement of an amputated or damaged part of the body controlled by various mechanisms and intended to perform specific actions and/or recreate the user's limb appearance.

User means a person or object who uses the prosthesis to perform specific functions.

The module is an individual part of the upper limb prosthesis design performing a specific number of functions.

The drive means a mechanism to transmit rotation from one place to another, including at least one motor and one reducer or a reducer motor, transmission, and attachment nodes.

### STATE OF ART

Document US 2005/0021154 A1 discloses a drive device for a finger prosthesis of substantially natural size designed to bend the finger prosthesis about a shaft relative to a fixing e.g. in a human or artificial metacarpus. The drive device comprises a motor which can be connected to an energy source e.g. a battery, and a transmission intended to transform a force from the motor to the finger prosthesis to perform the movement. The motor and transmission are placed in the finger prosthesis so that the bending shaft is contained in the finger prosthesis or in its extension at the fixing.

ABADJIEV VALENTIN ET AL: "Some principles of mathematical modelling and computer synthesis of hyperboloid gears with a conjugate linear tooth contact", 2014 INTERNATIONAL CONFERENCE ON INFORMATION SCIENCE, ELECTRONICS AND ELECTRICAL ENGINEERING, IEEE, vol. 1, 26 April 2014 (2014-04-26), pages 265-270, XP032672690, DOI: 10.1109/INFOSEEE.2014.6948111, ISBN: 978-1-4799-3196-5, relates to designing mathematical models, oriented to the synthesis of gear sets with skewed axes.

Document CN 107 803 849 A discloses a robot finger. In order to enable the robot finger to be more convenient, three motors are designed to separately control the robot finger of three joints. The motors are separately installed in the fingers adjacent to the joint, a micro motor is used as a power mechanism, a turbo-worm is used as a transmission mechanism, and the power mechanism is closely connected with the transmission mechanism and a joint movable part. The axial rotation of the motor is decelerated by virtue of a turbo-worm mechanism and changed to the vertical rotation so as to push the rear knuckle to rotate.

Document US 2013/0030550 A1 discloses a modular human hand prosthesis with modular, mechanically independent finger modules, which may be applied in prosthetics as a replacement of a single finger, a group of fingers or whole hand or arm, as well as in robotics as a gripper. The device has at least one mechanically independent finger module, particularly consisting of a fixed finger base, a finger proximal segment, a finger distant segment, a rod and a rotational drive; the finger base is equipped with a rod rotational proximal joint and the rotational joint of the finger proximal segment is connected via the rod proximal joint, rod, the rod distant segment and at the same time via the proximal joint of the finger proximal segment, the finger proximal segment, the distant joint of the finger proximal segment with the finger distant segment, and at the same time the distant joint of the finger proximal segment constitutes the rotational permanent centre of the drive.

Document US 2010/0005918 A1 discloses a joint mechanism that includes a first link, a second link, a third link, a shaft supported on the second link to be rotatable about an axis thereof, and movable in an axis direction thereof, a first worm and a second worm mounted on the shaft, a first worm wheel in mesh with the first worm to pivotally move the first link with respect to the second link, and a second worm wheel in mesh with the second worm to pivotally move the third link with respect to the second link. The first link and the third link are pivotally moved in opposite directions to each other by rotating the shaft, and pivotally moved in identical directions to each other by moving the shaft in the axis direction.

Bio-electrical prostheses are known in the prior art, which implement digit member mobility functions using various methods.

The claimed technical result is achieved by design used for movement transmission into a single part, which makes it possible to fix the separate motor of the digit member either in a housing of the digit member or within a wrist housing. Each digit member has an individual, independent motor and drive, making it possible for the prosthesis to perform gestures and individual operations imitating natural movements of human fingers and wrist.

There are also prostheses where motors are used for simultaneous motion of two or four rigidly fixed digit members. Digit members are arranged in groups, making the motor simpler, lighter, and therefore convenient. The disadvantage of such technical solutions is the difficulties in manufacturing a compact prosthesis since the currently available solid motors limit the minimal possible size of the prosthesis digit member module. In addition, the mutual location of hinges used to attach digit members to the wrist prosthesis and possible gestures and motions that the prosthesis can perform are both limited.

Meanwhile, manufacturers from all over the world strive to make the prosthesis lighter and smaller so that its weight and size would correspond to that of the user's own wrist, and in this case, using the prosthesis does not result in spinal curvature, imbalance in the musculoskeletal system and is better perceived by the user. The inability to miniaturize the working mechanisms of the prosthesis without reducing the reliability and rigidity of the design hinders the development of pediatric prosthetics. Currently, there are no optimal shapes and design solutions that would allow making a prosthesis that completely imitates the size and functions of the human wrist, but this challenge remains highly relevant.

Modern prosthetics usually use simple mechanisms to transmit motion: cylindrical reducers with tie-rods driving to digit member blocks; screw-nut transmission with digit member tie-rods driving, and wire transmissions.

There are more advanced mechanisms of digit members in bio-electrical prostheses that use cylindrical worm reducers, such as patent No. U39387095B2 Prosthetics and orthotics, publication date: 2015-07-09. This solution reveals a digit member design where the drive is located in the digit member housing and protrudes beyond the worm ring. This drive arrangement allows to move the digit member independently and may provide a digit member size close to an adult's finger when using existing microsized motors. At the same time, the transmission in the wrist module attachment node is made in such a way that the overall wrist dimension does not correspond to the natural one: protruding elements on the wrist lead to unreasonable use of space and result in the increased digit member module dimensions or decrease in bearing capacity. Thus, the disadvantage of this technical solution is that this prosthetic wrist has either increased dimensions of digit member modules or the reduced bearing capacity, expressed in the impaired appearance of the wrist prosthesis or performance, and also complicates the use of such design for small-sized prostheses.

Spiroid reducers are also known in the prior art but are not applied for prostheses, including inventions No. SU 364783 (publication date: 1972-12-28), SU 209.167 publication date: 1968-01-17), SU 1054602 (publication date: 1983-1115), SU 1520278 (publication date: 1989-11-07), and others as well as specialized publications in this field (such as Kraynev A.F. Glossary for mechanisms. Moscow: Machine Engineering, 1987. p. 433; GOST 22850-77. Spiroid reducers. Terms, definitions, and designations, Moscow: Publisher of Standards, 1978).

### EMBODIMENT OF TECHNICAL SOLUTION

The invention is defined by independent claim 1.

Advantageous embodiments are defined by the dependent claims.

The technical result of the invention consists in providing a design of an upper limb prosthesis which is characterized by a compact size concerning the overall prosthesis assembly, a higher load capacity despite the smaller dimensions of the digital member, an aesthetic appearance, and protection of the gear mesh against ingress of foreign objects and environmental factors. The thumb must rotate around the longitudinal axis along with folding unlike other digit members having only one degree of freedom. The rotation capacity of other digit members is also not precluded but is not mandatory in terms of prosthesis users.

The solutions known in the prior art are based on using a worm reducer in digit member prosthesis modules. They require special design to provide the aesthetic appearance corresponding to the human finger size and protect the gear mesh against environmental factors. This complicates the design, increases weight and dimensions, complicates the re-use of digit member module design in small prostheses, and requires other compositions with technical indicators of the gear mesh quality such as the number of simultaneously engaged gear, and other design solutions. A worm reducer limits the methods of attaching the tie-rod connecting the lower phalange with the fixed part of the digit member module since the space above the lower phalange rotation axis where the tie-rod attachment is located due to geometrical conditions is occupied by a worm reducer or front support of the worm. Another disadvantage of the worm reducer is that the worm is exposed to significant radial and axial loads, thus requiring bearings capable of taking them simultaneously, while miniature rolling bearings usually have the low bearing capability in one direction. This makes it necessary to use the plain bearings as worm supports, which reduces the overall mechanism efficiency.

A spiroid reducer usage for digit member prosthesis has not been revealed in the prior art. It is not obvious for experts for the following reasons: no available standards for geometric synthesis and calculations of spiroid engagement parameters, process challenges in manufacturing a small-module spiroid gear mesh by running in at universal gear-cutters, no available methods, and software to build 3D electronic models of skid-type gear surfaces, challenges in manufacturing small-module spiroid reducers by processing at CNC machines.

The use of a spiroid reducer for wrist prostheses has not been revealed due to the above reasons, since manufacturing a wrist prosthesis with digit member modules in spiroid reducers requires calculation, designing, and manufacturing a small-module spiroid gear mesh.

The technical result of the invention consists of providing a design of an upper limb prosthesis characterized by a compact size with regard to the overall prosthesis assembly. At the same time, the small width of the digit member module around the lower phalange hinge allows the implementation of the small standard size of the wrist prosthesis. The attachment of the upper phalange inside the spiroid reducer space decreases the digit member module size. It provides an aesthetic appearance since the tie-rod hides in the housing of the lower phalange. The capability of simultaneous gear mesh of two spiroid gear rings with the worm ensures a higher loading capacity at lower dimensions since it doubles the number of engaged gears from 3-4 to 6-8, which increases the mechanism bearing capacity. The double-ring transmission can compensate for the radial forces on the worm resulting from engagement with two spiroid reducers. The rear worm support takes axial load only, making it possible to use a rolling bearing as the rear worm support. This allows the increasing bearing capacity of the mechanism without efficiency losses in the rear support. Moreover, since the teeth of spiroid reducers are oriented inside and can be seen only from edges, the prosthesis looks more aesthetic, and the gear mesh against ingress of foreign objects and environmental factors.

The claimed technical result is provided by integrating a spiroid gear sector and a bearing element used for transmitting motion to a single part - a combined lever or a combined ring reducer, which reduces the size and weight of transmission and allows attaching the lower phalange tie-rod in the spiroid reducer housing in the space within the spiroid gear reducer layout. Moreover, a digit member's motor can now be arranged either in a housing of the digit member or within a wrist housing.

The spiroid drive reducer can be used to make a new design of prosthesis digit members and also a new design of an upper limb prosthesis or its module.

Thumb modules with spiroid transmission implying location of the digit member rotation drive inside the mobile part of the digit member consists of an electrical reducer motor, a housing made of aluminum, its alloys, titanium or its alloys where at least one adaptor bushing is installed to attach the drive and secure the worm against axial movement; at the same time, the bushing can be made integral with the reducer motor housing, and a plain bearing as the rear worm support, a roller bearing with angular contact or a thrust roller bearing, a spiroid one- or multi-thread worm of cylindrical or conic type, front worm support in the form of a plain bearing or a roller bearing, left and right spiroid ring reducers made of stainless steel with wear-resistant coating, carbon steel with anti-corrosion coating, bronze, aluminum alloy with special coating or another material with similar properties installed in the housing on plain or roller bearings while they can be made integral with the bearing elements - for example, a protrusion for attachment holes and/or semi-axis. Combined rings can be connected, for example, by means of a bracket mated with the wrist prosthesis element. A decorative plate of the digit member is attached to the drive housing and can be made of polyamide, composite based on carbon fiber, rubber, silicone, and their combination. Thus, the digit member rotation mechanism is entirely located inside the digit member plate. The collaborative design of the spiroid ring reducer allows obtaining the thumb module width around the lower phalange rotation axis not exceeding 10-11 mm.

The digit member module with spiroid transmission implying actuator arrangement inside the palm of a prosthetic wrist consists of an electrical reducer motor, a housing made of aluminum, its alloys, titanium, or its alloys where at least one adaptor bushing is installed to mount the actuator and secure the worm against the axial shift, while the bushing can be integral with the reducer motor housing. The worm engaged with spiroid gear installed on a pair of combined levers drives a four-link lever mechanism of the digit member module housing, a pair of combined spiroid levers, a tie-rod, and the upper digit member phalange. Levers can be connected to each other and the plate, for example, using remote bushings, and are mounted on semi-axis inside the housing being integral with the lever parts, on the housing part, or axis being individual parts.

The plate can be made of plastic, carbon composite, and composite based on fiberglass, rubber, silicone, or combinations. The upper phalange is attached on the rotation axis to the combined levers of the lower phalange. It is connected with the housing part of the digit member module by a tie-rod, whereby the tie-rod and the digit member housing are coupled inside the spiroid reducer space. The collaborative design of the spiroid gear lever allows for the digit member module width in the area of the lower phalange rotation axis not exceeding 10-11 mm.

The wrist module with a spiroid reducer consists of an aluminum or titanium alloy frame made by casting, stamping, welding, or additive method. Modules of at least two digit members with spiroid drive reducers, a module of active or passive rotation of the thumb, to which a thumb module with a spiroid drive gearbox is attached, are mounted to the frame. Protective and decorative plates attached to the frame are made of, for example, carbon composite, composite based on glass fiber, polyamide, rubber, or their combination. This frame serves to attach at least one adapter to install the wrist prosthesis on the patient sleeve or couple with other modules such as wrist bend module, active or passive wrist rotation module, wrist prosthesis fast replacement module. Digit member motor control boards can be attached to the internal ribs of plates; a lower phalange position feedback magnetic sensor can be mounted on the boards; in this case, the board is located so that the lower phalange axis with an attached magnet opposes the sensor microchip and thus defines the absolute angular position of each digit member. The driver board with the sensor in the thumb drive can be attached inside the thumb plate. The use of digit member modules with spiroid reducer allows for wrist modules from 75 to 95 mm in the widest part of the wrist, without changing the gear mesh parameters, digit member drive housing parameters, and qualitative changes of digit member module designs. The combined design of the spiroid gear lever allows digit member modules to have small width around the lower phalange hinge and thus limits the minimum possible width of the wrist prosthesis and its overall dimensions. Prosthesis control is provided by signals of mio-electrical sensors located in the sleeve or another structural part of the prosthesis; a battery can also be located in the sleeve.

### SHORT DESCRIPTION OF DRAWINGS

This solution refers to figures that show as follows.

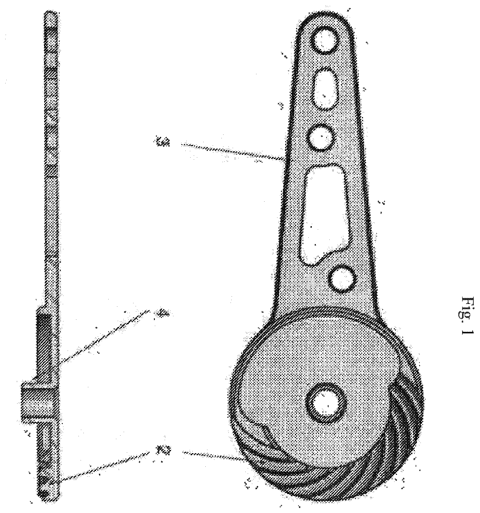

- Fig. 1 -: combined lever,

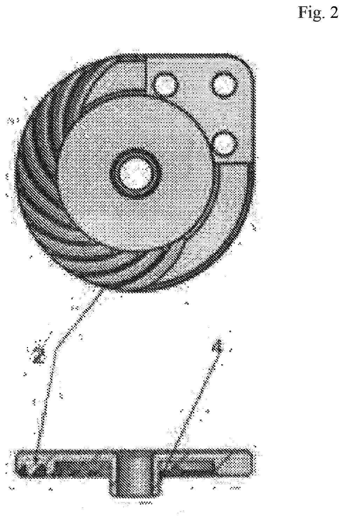

- Fig. 2 -: combined ring reducer,

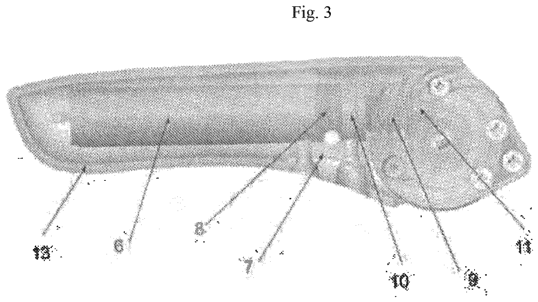

- Fig. 3 -: thumb module (side view),

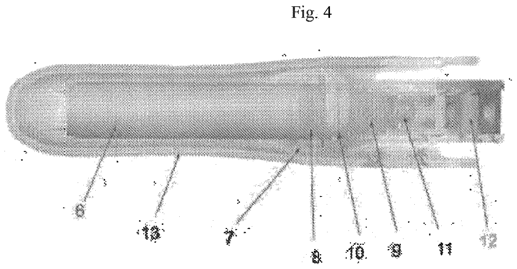

- Fig. 4 -: thumb module (top view),

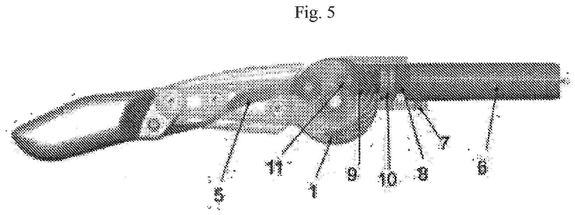

- Fig. 5 -: digit member module (side view),

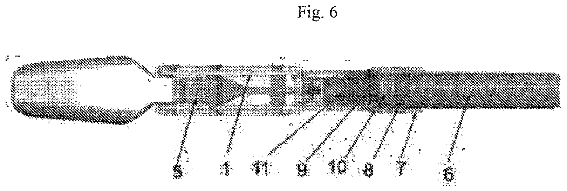

- Fig. 6 -: digit member module (top view),

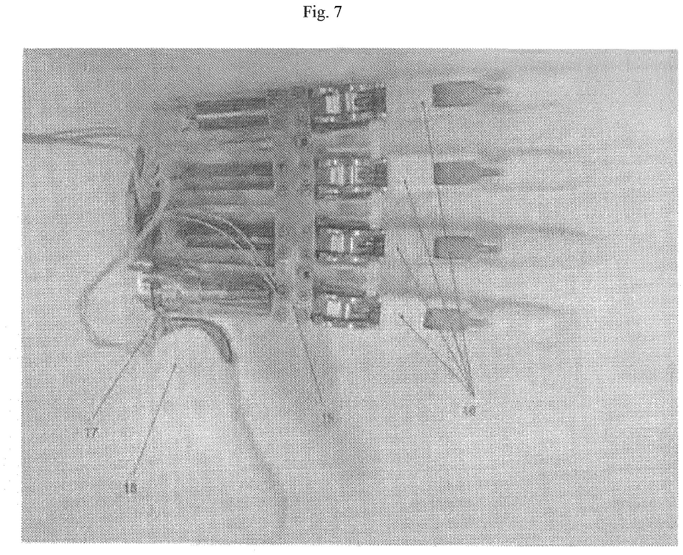

- Fig. 7 -: wrist module (general view).

1 - combined lever with spiroid reducer ring; 2 - spiroid gear sector; 3 - lever; 4 - semi-axis; 5 - tie rod of the lower phalange; 6 - reducer motor; 7 - housing; 8 - adapter bushing; 5 - worm; 10 - roller bearing; 11- plain bearing; 12 - bracket; 13 - thumb plate; 14 - rotation axis; 15 - wrist frame; 16 - digit member module with spiroid reducer; 17 - thumb rotation module; 18 - thumb module.

### EMBODIMENT OF TECHNICAL SOLUTION

A digit member module with a two-ring spiroid gear transmission was developed to solve the described problem. It includes a gear mesh component integrated into each part of a combined lever (1) - a spiroid gear sector (2), bearing elements of the part - a lever (3) and a semi-axis (4), which allowed attaching the tie rod of the lower phalange (5) in the spiroid transmission housing within the spiroid gear transmission layout. The spiroid transmission is made of two-ring and has two spiroid gear sectors (2) on two bearing parts (1), which also allows increasing the number of simultaneously engaged gears.

The technical result in embodiment of the invention is achieved as follows:
The thumb module with a spiroid drive reducer implying location of the digit member rotation module inside the movable part of the digit member consists of the electrical reducer motor (6), the housing (7) made of D16T alloy where the adapter bushing (8) is installed to attach the drive and secure the worm (9) against the axial shift, the rear worm support in the form of a thrust plain bearing (10), the cylindrical single-thread worm, the front worm support in the form of a plain bearing (11), left and right spiroid reducer rings made of stainless steel, installed in the housing on plain bearings and made integral with bearing elements - a protrusion for attachment holes and semi-axis. Combined ring reducers are connected by the bracket (12) coupled with the thumb rotation drive. The thumb plate (13) is attached to the housing drive and is made of polyamide.

The digit member module with a spiroid reducer implying the location of the wrist prosthesis drive inside the movable part of the palm consists of the electrical reducer motor (6), the housing (7) made of D16T alloy where the adapter bushing (8) is installed to attach the drive and secure the worm (9) against the axial shift, the rear worm support in the form of a thrust plain bearing (10), the cylindrical single-thread worm, the front worm support in the form of a plain bearing (11), left and right spiroid reducer rings made of stainless steel and installed in the housing on plain bearings and made integral with bearing elements - a lever (3) and a semi-axis (4). A tie-rod (5) of the stainless alloy is attached between spiroid gear levers inside the housing and connects the fixed housing (7) with the upper phalange. Levers are connected to each other and the lower phalange plate using remote bushings. The lower phalange plate is made of polyamide. The upper phalange is attached on the rotation axis (14) to combined levers of the lower phalange and is connected to the housing part using a tie-rod.

The wrist module with spiroid transmission of digit member modules consists of the frame (15) made of aluminum alloy by the additive method. Digit member modules with a spiroid drive reducer (16) and a thumb active rotation module (17), whereto the thumb module (13) is attached, mounted on the frame. Protective and decorative plates made of polyamide are attached to the frame.

## Claims

1. A digit member module (16) of an upper limb prosthesis, the digit member module (16) being **characterized by** a spiroid transmission, wherein a digit member rotation module is located inside a movable part of the digit member module (16), and wherein the spiroid transmission has at least one spiroid gear ring reducer on one of the sides from an axis of a worm (9), the spiroid gear ring reducer being included in a combined lever (1) having a spiroid gear sector (2) and bearing elements in the form of a lever (3) and a semi-axis (4).

2. The digit member module according to claim 1, **characterized in that** a thrust roller bearing (10) is used as the rear support of the worm (9).

3. An upper limb prosthesis including a digit member module (16) according to any of the previous claims, wherein the upper limb prosthesis is a wrist prosthesis, and the digit member module is attached to a palm of the wrist prosthesis.

4. The upper limb prosthesis according to claim 3, **characterized in that** the spiroid transmission has at least one spiroid gear ring reducer on one of the sides from the axis of the worm (9).

5. The upper limb prosthesis according to claim 3, **characterized in that** a thrust roller bearing (10) is used as the rear support of the worm (9).

6. The upper limb prosthesis according to claim 3, **characterized in that** a tie rod (5) of a lower phalange is mounted inside the spiroid transmission.

7. A wrist module having a frame (15), wherein at least one digit member module (16) according to any of claims 1 or 2 is attached on the frame (15).

8. The wrist module according to claim 7, **characterized by** the use of a motor control board with feedback sensors, which is located on a rotation axis of the digit member module (16) movable link.

## Patentansprüche

1. Fingermodul (16) einer Prothese für die oberen Gliedmaßen, wobei das Fingermodul (16) ein Spiroidgetriebe aufweist, wobei das Fingerrotationsmodul innerhalb eines beweglichen Abschnitts des Fingermoduls (16) angeordnet ist, und wobei das Spiroidgetriebe mindestens ein Spiroid-Reduziergetriebe auf einer Seite der Schneckenachse (9) aufweist, wobei das Spiroid-Reduziergetriebe in einem Kombihebel (1) enthalten ist, der ein Spiroid-Zahnsegment (2) und Stützelemente in Form eines Hebels (3) und einer halben Achse (4) aufweist.

2. Fingermodul nach Anspruch 1, **dadurch gekennzeichnet, dass** ein Axialrollenlager (10) als Endstück der Schnecke (9) verwendet wird.

3. Prothese für die oberen Gliedmaßen mit einem Fingermodul (16) nach einem der vorhergehenden Ansprüche, wobei die Prothese für die oberen Gliedmaßen eine Handgelenksprothese ist und das Fingermodul an einer Handfläche der Handgelenksprothese angebracht ist.

4. Prothese für die oberen Gliedmaßen nach Anspruch 3, **dadurch gekennzeichnet, dass** das Spiroid-Getriebe auf einer Seite der Schneckenachse (9) mindestens ein Spiroid-Zahnradgetriebe aufweist.

5. Prothese für die oberen Gliedmaßen nach Anspruch 3, **dadurch gekennzeichnet, dass** ein Axialrollenlager (10) als Endstück der Schnecke (9) verwendet wird.

6. Prothese für die oberen Gliedmaßen nach Anspruch 3, **dadurch gekennzeichnet, dass** eine Zugstange (5) eines unteren Fingergliedes im Inneren des Spiroid-Getriebes angebracht ist.

7. Handgelenkmodul mit einem Rahmen (15), wobei mindestens ein Fingermodul (16) nach einem der Ansprüche 1 oder 2 am Rahmen (15) befestigt ist.

8. Handgelenkmodul nach Anspruch 7, **gekennzeichnet durch** die Verwendung einer Motorsteuerplatte mit Rückkopplungssensoren, die auf einer Drehachse des beweglichen Gliedes des Fingermoduls (16) angeordnet ist.

## Revendications

1. Un module de membre digital (16) d'une prothèse de membre supérieur, le module de membre digital (16) ayant un engrenage spiroïdal, où un module de rotation de membre digital est situé à l'intérieur d'une partie mobile du module de membre digital (16), et où l'engrenage spiroïdal comporte au moins un réducteur à roue spiroïdale sur l'un des côtés d'un axe d'une vis sans fin (9), le réducteur à roue spiroïdale étant inclus dans un levier combiné (1) comportant un secteur de roue spiroïdale (2) et des éléments porteurs sous la forme d'un levier (3) et d'un demi-axe (4).

2. Le module de membre digital selon la revendication 1, **caractérisé en ce qu'**une butée à rouleaux (10) est utilisée comme extrémité arrière de la vis sans fin (9).

3. Une prothèse de membre supérieur comprenant un module de membre digital (16) selon l'une quelconque des revendications précédentes, où la prothèse de membre supérieur est une prothèse de poignet, et le module de membre digital est fixé à une paume de la prothèse de poignet.

4. Une prothèse de membre supérieur selon la revendication 3, **caractérisée en ce que** l'engrenage spiroïdal comporte au moins un réducteur à roue spiroïdale sur l'un des côtés de l'axe de la vis sans fin (9).

5. La prothèse de membre supérieur selon la revendication 3, **caractérisée en ce qu'**une butée à rouleaux (10) est utilisée comme extrémité arrière de la vis sans fin (9).

6. La prothèse de membre supérieur selon la revendication 3, **caractérisée en ce qu'**un tirant (5) d'une phalange inférieure est monté à l'intérieur de l'engrenage spiroïdal.

7. Le module de poignet comportant un cadre (15), dans lequel au moins un module de membre digital (16) selon l'une des revendications 1 ou 2 est fixé au cadre (15).

8. Le module de poignet selon la revendication 7, **caractérisé par** l'utilisation d'une carte de commande de moteur avec des capteurs de rétroaction, qui est située sur un axe de rotation de la liaison mobile du module de membre digital (16).
